# FASCICULE DE BREVET EUROPEEN

(11) **EP 0 759 287 B1**
(45) Date de publication et mention de la délivrance du brevet: **12.02.2003**
(21) Numéro de dépôt: 96401666.1
(22) Date de dépôt: 25.07.1996
(51) Int. Cl.: A61F 2/01

(54) **Filtre définitif comportant un orifice pour le passage de dispositifs médicaux et son procédé de fabrication**
Permanenter Filter mit einer Öffnung zum Durchführen von medizinischen Vorrichtungen und sein Herstellungsverfahren
Filter for permanent use having an opening for the passage of medical devices and method of manufacture

(30) Priorité: 10.08.1995 FR 9509736
(43) Date de publication de la demande: 26.02.1997
(73) Titulaire: B. BRAUN CELSA, 86360 Chasseneuil du Poitou (FR)
(72) Inventeur: Nadal, M. Guy, 86000 Poitiers (FR)
(74) Mandataire: Lerner, François

(56) Documents cités:
- FR-A- 2 573 646
- FR-A- 2 696 092
- US-A- 5 059 205

## Description

L'invention se rapporte à un filtre sanguin destiné à être introduit à l'intérieur d'un vaisseau du corps d'un patient, en particulier pour la retenue de caillots sanguins, afin d'éviter les risques d'embolie.

Dans les documents FR-A-2 573 646 et FR-A-2 696 092, le filtre décrit comporte une série de branches élastiques filiformes formant une structure naturellement tronconique adaptée pour être radialement resserrée ou expansée. Chacune des branches comprend en outre des moyens de fixation à la paroi du vaisseau, tels que des crochets. Le filtre est ainsi dit "à implantation définitive".

Bien que d'intérêt, un tel filtre présente cependant en pratique un inconvénient

En effet, lorsque le filtre a été implanté dans son vaisseau récepteur, il n'est plus possible d'accéder, par la voie d'accès utilisée pour l'introduire, à la partie du vaisseau se situant au-delà du filtre. Or, chez certains patients, il est parfois nécessaire d'intervenir justement dans cette zone, par exemple afin de retirer un premier filtre ayant été impanté dans un premier temps et de façon temporaire avant le filtre définitif, lorsque les risques d'embolie étaient supposés n'être que passagers, ou encore afin d'introduire, après l'implantation du filtre définitif, par exemple une prothèse pour le traitement d'une sténose survenue à cet endroit.

Actuellement, l'intervention dans cette zone du vaisseau n'est souvent possible que par voie chirurgicale, ou en changeant de voie d'accès, ce qui n'est pas toujours possible (obstacle anatomique).

L'objectif de l'invention est d'obtenir un filtre à implantation définitive ayant d'excellentes qualités filtrantes et évitant l'inconvénient cité précédemment.

A cet effet, le filtre selon l'invention se caractérise en ce qu'il est traversé par un orifice s'étendant suivant l'axe du filtre et qui, à l'endroit de la zone de réunion des branches du filtre, est défini par une structure. sensiblement annulaire coaxiale au filtre et de diamètre variable.

De préférence, le diamètre de cet orifice sera compris entre 1,4 mm et 3 mm environ pour permettre le passage à travers lui de dispositifs ou appareils médicaux (notamment un filtre temporaire), ses dimensions permettant en outre de concilier une bonne capacité de filtration sanguine, et une introduction du filtre dans le vaisseau par un cathéter de petit diamètre.

A ce sujet, il paraissait raisonnable de considérer que plus on réduirait le diamètre de l'orifice, plus on pourrait utiliser un cathéter introducteur de faible diamètre, et plus on pourrait parallèlement avoir une efficacité de filtration accrue. Or, paradoxalement, le demandeur a découvert qu'il était préférable d'augmenter le diamètre de l'orifice du filtre jusqu'à des valeurs comprises donc entre 1,4 mm et 3 mm et de préférence 1,7 mm et 2,5 mm, ceci ne gênant en rien l'efficacité de la filtration et ne remettant pas en cause les conditions de l'introduction, puisqu'il est encore possible d'utiliser un cathéter introducteur de diamètre raisonnable.

L'état élargi pourra être obtenu soit lors de l'expansion du filtre dans le vaisseau, auquel cas il demeurera dans cet état dans le vaisseau, soit lors du passage d'un dispositif médical quelconque à travers le filtre expansé ou non, le diamètre de l'orifice retrouvant un état plus resserré après le passage du dispositif.

Une description plus détaillée de l'invention va maintenant être donnée en référence aux dessins annexés dans lesquels :
- les figures 1 à 5 représentent schématiquement différents modes de réalisation particuliers d'un filtre selon l'invention,
- les figures 6 à 8 montrent schématiquement les différentes étapes d'un procédé de fabrication du filtre selon l'invention,
- la figure 9 représente schématiquement un filtre destiné à être implanté temporairement,
- les figures 10 à 16 montrent schématiquement comment le filtre selon l'invention permet le retrait d'un filtre temporaire implanté antérieurement

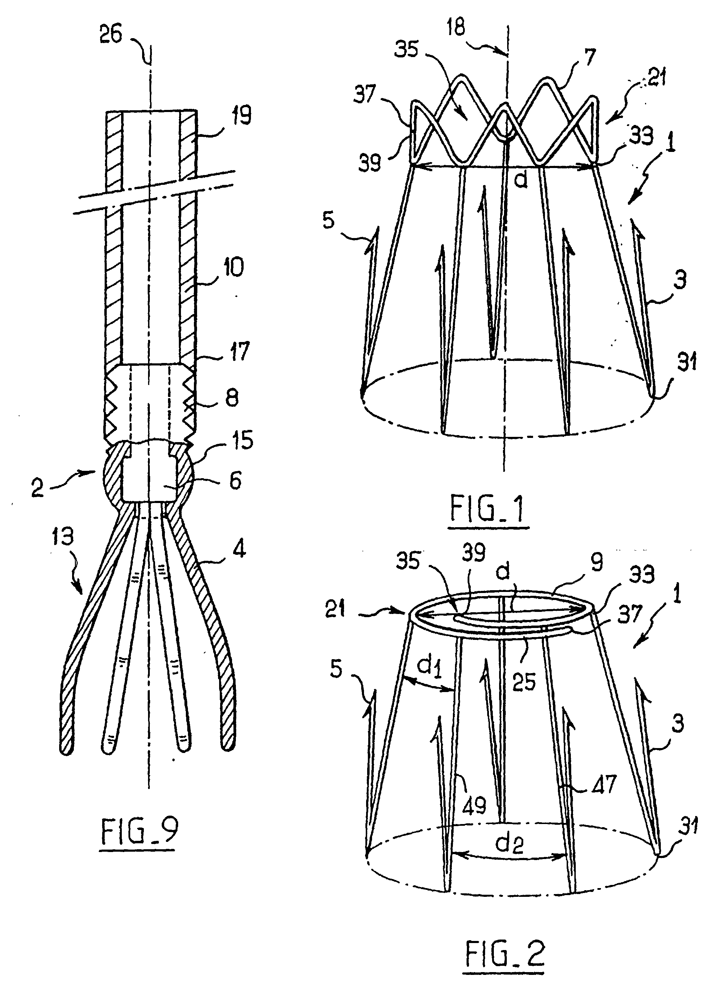

En se référant à la figure 1, on remarque que le filtre selon l'invention, repéré dans son ensemble 1, et que l'on appellera "filtre définitif" dans la suite de la description, comporte une série de branches allongées 3 ayant chacune une forme sensiblement en "V". Une extrémité libre de chaque "V" comporte un crochet 5 pour l'ancrage du filtre à la paroi du vaisseau. L'autre extrémité est reliée à une structure de liaison 21 annulaire, d'axe confondu avec celui du filtre. La zone 21 consiste ici en un fil métallique, par exemple en acier spécial tel que "phynox" (marque déposée) présentant une succession d'ondulations (en l'espère des zigzags) formant une structure annulaire élastique 7 définissant intérieurement l'orifice central 35 dont le diamètre (d) peut être plus ou moins resserré, et qui est peut être adapté pour permettre le passage à travers lui par exemple d'un filtre temporaire tel que décrit ci-après. Les différentes branches 3 peuvent être réalisées sous la forme de fins fils ou de lamelles métalliques en "phynox" (marque déposée). Elles ont chacune une extrémité distale 31 (correspondant à l'extrémité la plus éloignée du lieu d'introduction lorsque le filtre est en place dans le vaisseau) et une extrémité proximale 33 (extrémité la plus proche du lieu d'introduction) par laquelle elles sont fixées (soudées) à la structure annulaire 7.

La figure 2 représente une autre forme possible de réalisation d'un filtre 1 selon l'invention. Il s'agit d'une structure filamentaire dont les différentes branches 3 sont liées par leur extrémité proximale 33 à un fil (ou lame) métallique enroulé(e) sur lui(elle)-même suivant l'axe du filtre et formant un anneau 9 de type "ressort de montre", c'est-à-dire en spirale, dont l'orifice central présente un diamètre variable pouvant également être resserré ou écarté.

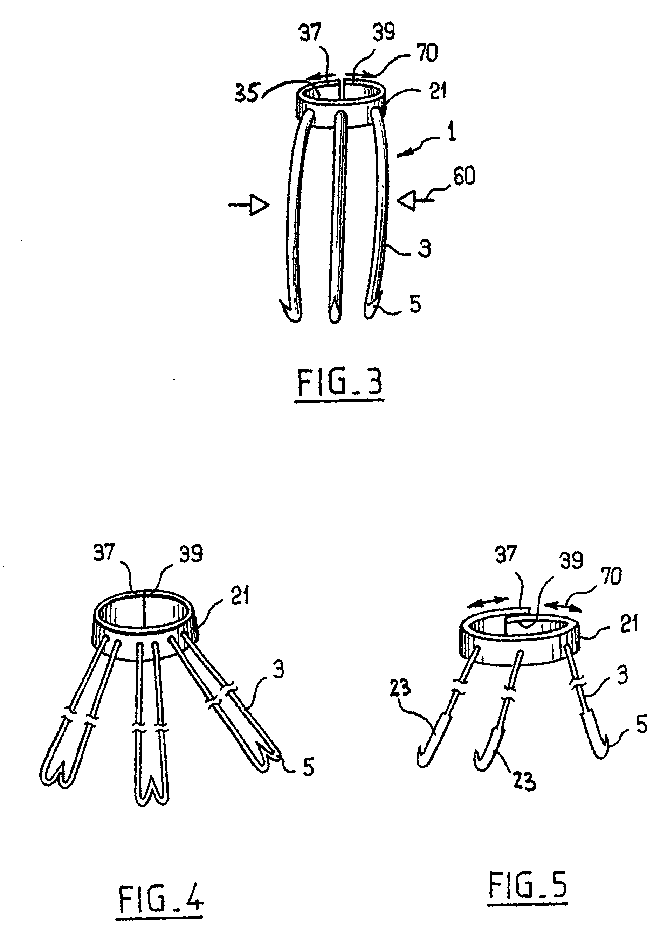

Au lieu de présenter une forme en "V", les différentes branches 3 pourraient consister, par exemple, en des pattes allongées sensiblement rectilignes (comme représenté sur la figure 3) ou en des fils recourbés sur eux-mêmes en épingle à cheveu et terminés par des crochets (figure 4), ou encore en des fils terminés par une portion en plaque, 23, comportant des crochets (figure 5).

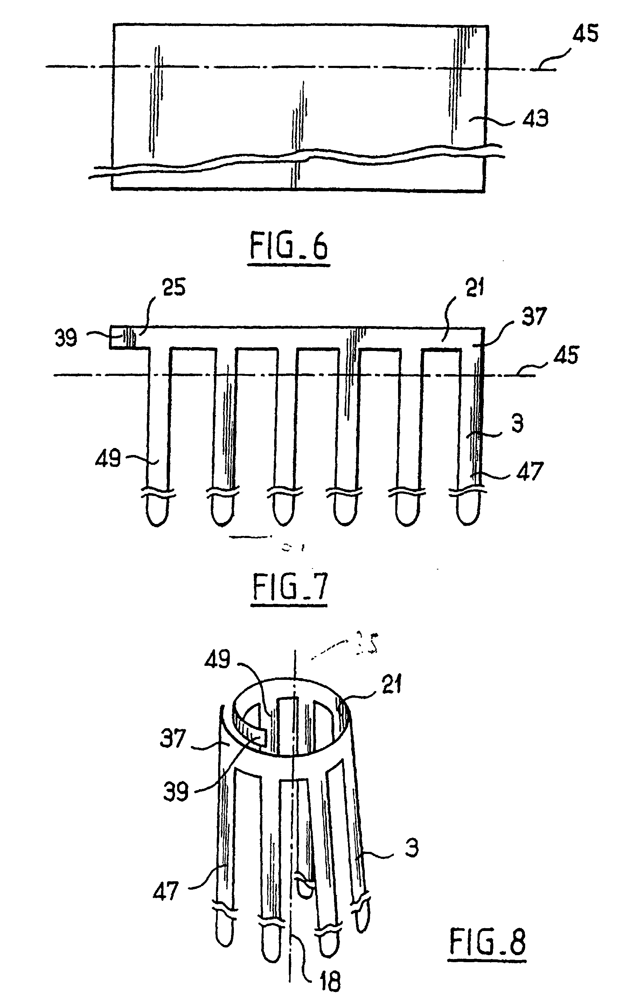

Le filtre selon l'invention peut être fabriqué en une seule pièce, comme schématisé aux figures 6 à 8. Une fine plaque sensiblement plane déformable 43, par exemple en "phynox" (marque déposée) d'environ 0,08 à 0,20 mm d'épaisseur, est découpée de façon à obtenir une série de branches 3 s'étendant les unes à la suite des autres le long d'un axe 45 appartenant au plan de la plaque 43, dirigées chacune transversalement par rapport à cet axe 45 et comprenant une première et une dernière branches, respectivement 47 et 49.

Les branches sont écartées les unes des autres sensiblement par une même distance (d₁), sauf lesdites première et dernière branches entre elles, qui sont écartées l'une de l'autre d'une distance (d₂) supérieure à (d₁) (au moins dans l'état radialement déployé du filtre), ceci permettant d'éviter à ces deux branches de se chevaucher.

Une bande de liaison 21 relie les branches 3 les unes aux autres et a une longueur au moins égale à la distance qui sépare la première branche 47 de la dernière, 49.

Le filtre est ensuite obtenu en recourbant la bande 21 de façon à rapprocher ses deux extrémités opposées 37, 39 situées au voisinage respectivement des première et dernière branches 47, 49, de façon à définir un orifice central 35 ayant un diamètre (d) compris entre 1,4 mm et 3 mm environ. Dans l'état radialement resserré du filtre, ces deux extrémités 37, 39 peuvent venir bord à bord ou non.

La zone de liaison 21 peut comporter une portion dépourvue de branches 3, pour former une languette 25 qui permet un recourbement aisé de la bande 21 sans chevauchement des branches, dans le cas où les deux extrémités opposées 37, 39 ne sont pas reliées l'une à l'autre (figures 7 et 8).

Le filtre 1 pourrait également être obtenu par emboutissage, découpage au laser ou découpage électrochimique d'une plaque métallique, telle que 43.

Il pourrait également être fabriqué en plusieurs pièces, en découpant, dans la plaque 43, une bande que l'on courberait sur elle-même pour rapprocher ses extrémités opposées, en définissant ainsi l'orifice central.

On fixerait par ailleurs sur cette bande une série de structures allongées définissant les branches du filtre.

Une manière de procéder identique peut être envisagée à partir d'un fil de structure, tel qu'un fil métallique, rectiligne ou présentant des ondulations (comme le fil 7 de la figure 1).

Pour son introduction dans le vaisseau, le filtre définitif 1 selon l'invention adopte un état contraint dans lequel les branches 3 sont rapprochées de l'axe 18 du filtre jusqu'à lui être sensiblement parallèles. Lorsqu'il est en place dans le vaisseau, le filtre adopte un état déployé, les branches 3 s'écartant alors de l'axe 18, et les crochets 5 s'ancrant dans la paroi du vaisseau 11.

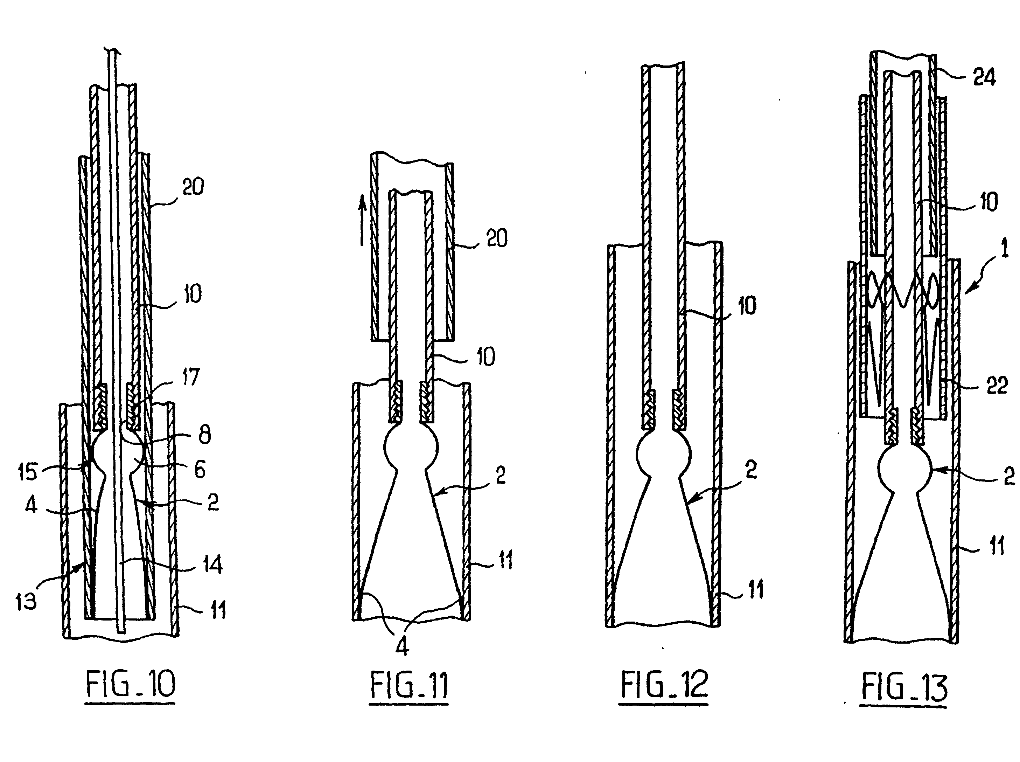

La figure 9 représente un filtre, répéré dans son ensemble 2, destiné à être implanté dans un vaisseau 11 pendant une période temporaire. Ce filtre sera appelé "filtre temporaire" dans la suite de la description.

Il peut s'agir du filtre décrit dans FR-A-2 666 980.

Ce filtre comprend une structure élastique ayant une extrémité distale 13 formée de plusieurs pattes 4, réunies entre elles au niveau d'une extrémité proximale 15 et comprenant un prolongement 8 relié à l'extrémité distale 17 d'une tige-support 10.

La tige-support 10 a de préférence une longueur au moins égale à la distance séparant la zone d'introduction du filtre et de ses moyens d'implantation à travers la peau du patient de la zone vasculaire d'implantation dudit filtre. De cette façon, il sera aisé de retirer le filtre 2 à la fin de sa période d'implantation, en tirant sur la tige-support 10 qui sera de préférence un tube creux ou un cathéter.

Le filtre temporaire 2 schématisé comporte en outre un orifice central 6 coaxial au tube 10, permettant son glissement le long d'un fil de guidage 14.

La figure 10 représente schématiquement l'introduction du filtre 2, qui se trouve pour le moment dans son état radialement resserré dans un premier cathéter 20. L'ensemble est glissé dans le vaisseau 11, le long du fil de guidage 14 jusqu'à la zone d'implantation vasculaire, après une ponction percutanée (appelée zone d'introduction) via un introducteur non représentée (méthode dite de "SELDINGER").

Pour positionner le filtre dans le vaisseau, il suffit alors, comme représenté à la figure 11, de déplacer en translation relative le cathéter 20 et le filtre 2, afin de déployer les pattes 4, qui viennent prendre appui sur la paroi du vaisseau 11.

La figure 12 représente le filtre 2 dans sa position déployée dans le vaisseau 11.

La figure 13 montre comment le filtre définitif 1 est introduit, chez des patients ayant bénéficié de l'implantation d'un filtre temporaire, mais pour lesquels les risques d'embolie se prolongent dans le temps, contrairement à ce que l'on attendait.

Le filtre 1 présente un diamètre réduit, dans un second cathéter 22, dans lequel est inséré un troisième cathéter 24. L'ensemble est glissé dans le vaisseau 11 jusqu'à l'emplacement vasculaire d'implantation du filtre temporaire 2, par la même voie d'accès percutanée que le filtre 2.

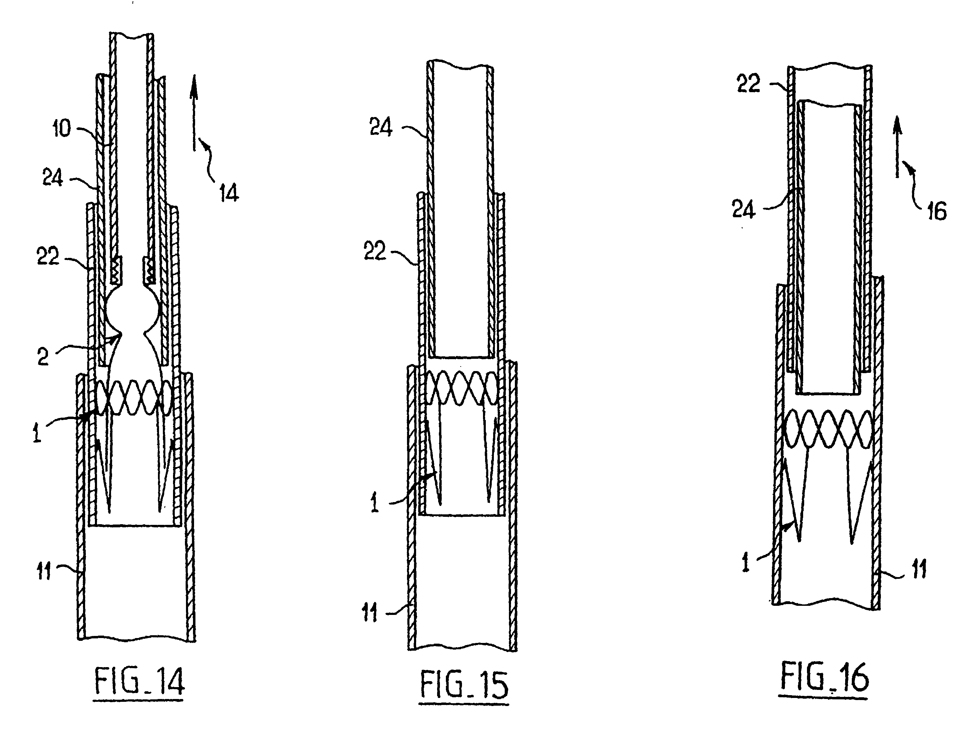

La figure 14 montre schématiquement le retrait du filtre 2. Lorsque le chirurgien tire sur le tube-support 10 dans le sens de la flèche 14, le filtre 2 pénètre dans le cathéter 22, puis traverse le filtre définitif 1 en élargissant si nécessaire sur son passage le diamètre de l'orifice central de ce dernier. Le filtre 2 est ensuite glissé dans le cathéter 24, avant d'être retiré entièrement hors du corps du patient. Après le passage du filtre temporaire 2, le diamètre de l'orifice central du filtre définitif retrouve sa position resserrée. Il pourrait cependant également rester élargi.

Le filtre définitif est ensuite introduit dans le vaisseau en déplaçant le cathéter 22 dans le sens de la flèche 16, en translation relative par rapport au cathéter 24. Les pattes 3 sont déployées et s'accrochent à la paroi du vaisseau 11 par l'intermédiaire des crochets 5.

Les mêmes résultats pourraient également être obtenus en procédant à l'implantation du filtre définitif avant le retrait du filtre temporaire.

## Revendications

1. Filtre sanguin (1) destiné à être introduit à l'intérieur d'un vaisseau (11) du corps d'un patient, en particulier pour la retenue de caillots sanguins, ledit filtre présentant un axe (18) et comportant plusieurs branches (3) ayant chacune une extrémité distale (31) et une extrémité proximale (33), lesdites branches (3) étant reliées entre elles par une structure de liaison (7, 9, 21) traversée par un orifice axial (35) et comportant des moyens de fixation (5) à la paroi du vaisseau (11) **caractérisé en ce que** la structure de liaison (7, 9, 21) qui définit ledit orifice (35) est élastiquement déformable entre un premier diamètre resserré et un deuxième diamètre élargi.

2. Filtre selon la revendication 1, **caractérisé en ce que** le diamètre dudit orifice axial (35) est compris entre 1,4 mm et 3 mm, et de préférence 1,7mm et 2,5 mm environ.

3. Filtre selon l'une des revendications 1 ou 2, **caractérisé en ce que** la structure de liaison (7,9,21) présente deux extrémités opposées (37, 39) rapprochées l'une de l'autre pour définir une forme annulaire.

4. Filtre selon la nevendication 3, **caractérisé en ce que** les deux extrémités opposées (37, 39) de la structure de liaison sont disjointes.

5. Filtre selon l'une quelconque des revendications précédentes, **caractérisé en ce que** la structure de liaison (7,9,21) présente une languette (25) dépourvue de branches (3).

6. Filtre selon l'une quelconque des revendications 1 à 5, **caractérisé en ce que** ladite structure de liaison (9, 21) est constituée par une structure enroulée sur elle-même, autour de l'axe (18) du filtre (1).

7. Filtre selon l'une des revendications 1 à 5, **caractérisé en ce que** ladite structure de liaison (7,21) est constituée par une structure en zigzag enroulée autour de l'axe (18) du filtre.

8. Procédé de fabrication d'un filtre sanguin (1) destiné à être introduit à l'intérieur d'un vaisseau (11) du corps d'un patient, **caractérisé en ce que**:
- on découpe dans une plaque sensiblement plane déformable (43) une série de branches (3) s'étendant les unes à la suite des autres le long d'un axe (45) appartenant au plan de la plaque (43), chacune dirigée transversalement par rapport à cet axe (45), comprenant une première et une dernière branches (47, 49) et reliées entre elles par une bande de liaison (21) ayant deux extrémités opposées (37, 39) situées au voisinage desdites première et dernière branches (47, 49), et
- on courbe ladite bande (21) pour rapprocher lesdites extrémités opposées (37, 39) en définissant ainsi un orifice central (35).

9. Procédé de fabrication d'un filtre sanguin (1) destiné à être introduit à l'intérieur d'un vaisseau (11) du corps d'un patient, **caractérisé en ce que**:
- on découpe, dans une plaque sensiblement plane déformable (43) et suivant un axe (45) de cette plaque, une bande (21),
- on courbe sur elle-même cette bande pour rapprocher ses extrémités (37, 39) en définissant ainsi un orifice central (35), et
- on fixe sur ladite bande (21), sensiblement perpendiculairement à l'axe (45) de la découpe, une série de structures allongées définissant pour le filtre des branches (3) expansibles radialement par rapport audit axe de découpe (45).

10. Procédé de fabrication d'un filtre sanguin (1) destiné à être introduit dans un vaisseau (11), **caractérisé en ce que** :
- on part d'une structure (7, 9, 21) filamentaire ou en plaque sensiblement rectiligne ou présentant des ondulations et enroulée sur elle-même pour constituer sensiblement un anneau présentant un axe (18),
- et on fixe à cette structure enroulée, sensiblement parallèlement à son axe, une série de structures allongées (3, 47, 49) définissant pour le filtre, des branches (3) expansibles radialement audit axe.

## Patentansprüche

1. Blutfilter (1) zum Einführen in das Innere eines Gefäßes (11) des Körpers eines Patienten, insbesondere für das Zurückhalten von Blutgerinnseln, wobei der Filter eine Achse (18) aufweist und mehrere Schenkel (3) beinhaltet, von denen jeder ein distales Ende (31) und ein proximales Ende (33) hat, wobei die Schenkel miteinander durch einen Verbindungsaufbau (7, 9, 21) verbunden sind, der von einer axialen Öffnung (35) durchquert wird, und an der Gefäßwand (11) Befestigungsmittel (5) aufweisen, **dadurch gekennzeichnet, daß** der Verbindungsaufbau (7, 9, 21), welcher die Öffnung (35) bestimmt, elastisch zwischen einem ersten verengten Durchmesser und einem zweiten erweiterten Durchmesser verformbar ist.

2. Filter nach Anspruch 1, **dadurch gekennzeichnet, daß** der Durchmesser der axialen Öffnung (35) zwischen etwa 1,4 mm und 3 mm und vorzugsweise etwa 1,7 mm und 2,5 mm beträgt.

3. Filter nach einem der Ansprüche 1 oder 2, **dadurch gekennzeichnet, daß** der Verbindungsaufbau (7, 9, 21) zwei entgegengesetzte Enden (37, 39) aufweist, die einander nahegebracht sind, um eine Ringform zu bilden.

4. Filter nach Anspruch 3, **dadurch gekennzeichnet, daß** die zwei entgegengesetzten Enden (37, 39) des Verbindungsaufbaus getrennt sind.

5. Filter nach einem der vorhergehenden Ansprüche, **dadurch gekennzeichnet, daß** der Verbindungsaufbau (7, 9, 21) eine Zunge (25) ohne Schenkel (3) aufweist.

6. Filter nach einem der Ansprüche 1 bis 5, **dadurch gekennzeichnet, daß** der Verbindungsaufbau (9, 21) aus einem Aufbau besteht, der auf sich selbst um die Achse (18) des Filters (1) eingerollt ist.

7. Filter nach einem der Ansprüche 1 bis 5, **dadurch gekennzeichnet, daß** der Verbindungsaufbau (7, 21) aus einem Zickzack-Aufbau besteht, der um die Achse (18) des Filters eingerollt ist.

8. Verfahren zum Herstellen eines Blutfilters für das Einführen in das Innere eines Gefäßes (11) des Körpers eines Patienten, **dadurch gekennzeichnet, daß**:
man in einer im wesentlichen ebenen, verformbaren Platte (43) eine Reihe von Schenkeln (3) ausschneidet, die sich einer nach dem anderen entlang einer Achse (45) erstrecken, die zu der Ebene der Platte (43) gehört, wobei jeder quer bezüglich dieser Achse (45) gerichtet ist, mit einem ersten und einem letzten Schenkel (47, 49), die miteinander durch ein Verbindungsband (21) verbunden sind, welches zwei entgegengesetzte Enden (37, 39) hat, die in der Nähe der ersten und letzten Schenkel (47, 49) liegen, und
man das Band (21) krümmt, um die entgegengesetzten Enden (37, 39) anzunähern, wobei so eine zentrale Öffnung (35) bestimmt wird.

9. Verfahren zur Herstellung eines Blutfilters (1) für das Einführen in das Innere eines Gefäßes (11) des Körpers eines Patienten, **dadurch gekennzeichnet, daß**:
man in einer im wesentlichen ebenen, verformbaren Platte (43), einer Achse (45) dieser Platte folgend, ein Band (21) abschneidet,
man das Band auf sich selbst biegt, um seine Enden (37, 39) einander näher zu bringen, wobei so eine zentrale Öffnung (35) bestimmt wird, und
man auf dem Band (21) im wesentlichen senkrecht zu der Schnittachse (45) eine Reihe von verlängerten Aufbauten befestigt, welche für den Filter Schenkel (3) bilden, die radial bezüglich der Schnittachse (45) ausdehnbar sind.

10. Verfahren zum Herstellen eines Blutfilters (1) für das Einführen in ein Gefäß (11), **dadurch gekennzeichnet, daß**:
man von einem fadenartigen Aufbau (7, 9, 21) oder einem Aufbau aus einer im wesentlichen rechteckigen Platte oder von einem Aufbau ausgeht, der Wellenlinien aufweist und auf sich selbst gerollt ist, um im wesentlichen einen Ring zu bilden, der eine Achse (18) aufweist,
und man an diesem eingerollten Aufbau im wesentlichen parallel zu seiner Achse eine Reihe von länglichen Aufbauten (3, 47. 49) befestigt, die für den Filter radial zu der Achse ausdehnbare Schenkel (3) bilden.

## Claims

1. A blood filter adapted to be introduced in a blood vessel of a patient body, especially for retaining blood clots, said blood filter having an axis (18) and comprising a plurality of legs (3) having a distal end (31) and a proximal end (33), the legs (3) being connected together by a connecting structure (7, 9, 21) having an axial hole (35) therethrough and comprising means (5) for fixing the blood vessel to the wall (11) of the vessel, **characterized in that** the connecting structure (7, 9, 21) which defines the hole (35) is elastically deformable between a first restricted diameter and a second enlarged diameter.

2. The blood filter according to claim 1 **characterized in that** the diameter of the axial hole (35) is comprised between 1, 4 mm and 3 mm, and is comprised advantageously between 1, 7 mm and 2,5 mm.

3. The blood filter according to claim 1 or claim 2, **characterized in that** the connecting structure (7, 9, 21) has two opposed ends (37, 39) disposed close to each other, for defining an annular shape.

4. The blood filter according to claim 3, **characterized in that** the two opposed ends (37, 39) of the connecting structure are disjointed.

5. The blood filter according to anyone of the preceding claims, **characterized in that** the connecting structure has an extension (25) free of any leg (3).

6. The blood filter according to anyone of claims 1 to 5, **characterized in that** the connecting structure is rolled up about itself, round the axis (18) of the blood filter (1).

7. The blood filter according to anyone of claims 1 to 5, **characterized in that** the connecting structure (7, 21) is comprised of a zigzag structure rolled up round the axis (18) of the filter.

8. A method for manufacturing a blood filter (1) adapted to be introduced in a blood vessel (11) of a patient body, **characterized by** the steps of:
- cutting in a deformable, substantially flat plate (43), a series of legs (3) extending side by side along an axis (45) belonging to the plane of the plate (43), every leg being orientated transversal to the axis (45), the legs comprising a first leg and a last leg (47,49) and being connected to one another by a connecting band (21) having two opposed ends (37, 39) located in the vicinity of said first and second legs (47, 49), respectively, and
- bending said band (21) for disposing said opposed ends (37, 39) close to each other, so that a central hole (35) is defined .

9. A method for manufacturing a blood filter (1) adapted to be introduced in a blood vessel (11) of a patient body, **characterized by** the steps of:
- cutting a band (21) in a deformable, substantially flat plate (23), along an axis (45) of said plate,
- bending on itself the band for disposing its ends (37, 39) close each other, so that a central hole (35) is defined, and,
- fixing to the band (21), substantially perpendicular to the axis (45) of the cut, a series of elongated structures defining legs (3) for the blood filter, said legs being expandable radially to the axis of cutting (45).

10. A method for manufacturing a blood filter (1) adapted to be introduced in a blood vessel (11), **characterised by** the steps of:
- obtaining a filamentary structure (9) or a substantially rectilinear plate structure (21) or an onduleted structure (7) rolled up about itself for substantially defining a ring having an axis (18),
- fixing to said rolled structure, substantially parallel to said axis, a series of elongated structures (3, 47, 49) defining legs (3) for the filter, said legs being expandable radially to said axis.
